# EUROPEAN PATENT APPLICATION

(11) **EP 1 333 097 A2**
(43) Date of publication of application: **06.08.2003**
(21) Application number: 03250450.8
(22) Date of filing: 24.01.2003
(51) Int. Cl.: C12Q 1/04, C12Q 1/42, C12N 9/52

(54) **Polyols in bioluminescence assays**

(30) Priority: 01.02.2002 GB 0202421
(71) Applicant: Celsis International PLC, Newmarket, Suffolk CB8 7SG (GB)
(72) Inventor: Foote, Nicholas Peter Martin, Kings Court, Newmarket, Suffolk CB8 7SG (GB); Kyle, Nigel, Celsis International plc, Newmarket, Suffolk CB8 7SG (GB); Thomas, Brian, Celsis International plc, Newmarket, Suffolk CB8 7SG (GB)
(74) Representative: Perry, Robert Edward

(57) **Abstract**

A method for the detection of microorganisms in a sample by bioluminescence following extraction of microbial ATP, comprises adding a polyol before or during the extraction.

## Description

### Introduction

A wide variety of industrial products and other samples involved in industrial processes (raw materials, in-process samples, environmental samples etc.) need to be tested for microbial contamination. In some cases the final product must be sterile; in other cases a limit is set on the total number of micro-organisms allowed. Often tests are performed for the presence of certain specific organisms, and again the requirement may be absence in a particular amount of sample or there may be a limit on the number allowed.

Traditionally these tests involve use of an agar plate. Micro-organisms are dispersed in or on the agar and the plate is then incubated until colonies appear, each colony indicating the presence initially of a single culturable micro-organism or a clump of organisms. Generally the maximum amount of material tested with an agar plate is limited to 1 millilitre unless the sample can be filtered; if greater sensitivity is required it must be enriched before an agar plate is used. Samples of foods and some drinks are often simply incubated at elevated temperature for a period of time in order to allow any contaminating organisms to multiply, and the incubated sample is tested with an agar plate. Other types of samples are dispersed in a nutrient medium for the incubation stage.

These traditional testing methods are slow. It usually takes at least 24 hours before healthy, fast-growing bacteria or yeasts form colonies large enough to comfortably count on an agar plate. However, many samples will contain stressed micro-organisms which need a recovery period before they begin to multiply, or organisms (including moulds) which grow slowly on common types of agar. Therefore most validated testing methods require an incubation period of no less than 2 days and in many cases 5 days or more.

Many microbiological tests can now be performed much more rapidly with the use of ATP bioluminescence. This makes use of the luciferase enzyme derived from firefly tails. A bioluminescence reagent contains luciferase with, *inter alia*, its substrate luciferin, magnesium ions and a suitable buffer. When adenosine-5'-triphosphate (ATP) is added to this reagent, luciferase catalyses the emission of light. ATP is an essential part of energy metabolism and therefore an indicator of the presence of living organisms or other organic matter.

Commercially-available kits designed for detecting the presence of living micro-organisms generally use the following protocol, or a variation of it.
1. A small sub-sample (e.g. 0.05 ml) is placed in a cuvette.
2. If the sample is expected to contain a significant amount of non-microbial ATP, a reagent containing an ATPase enzyme is added and the mixture is left for at least 5, and normally 15, minutes. The ATPase is often formulated with a detergent or other substance that will release the ATP from somatic cells but not from micro-organisms.
3. A microbial extractant is then added and allowed 10-30 seconds to release ATP from micro-organisms.
4. The bioluminescence reagent is added and the light emitted is measured for a period of a few seconds.

The result is recorded as an RLU (relative light unit) value. The higher the RLU value, the more micro-organisms were present in the sample.

There are many examples in the literature of studies which aim to optimise the formulation of bioluminescence reagent and of experiments comparing the performance of different extractant formulations. Far less effort has been put into optimising the formulation of the ATPase reagent.

When developing a kit for a particular application, the chosen ATPase activity tends to be a compromise. Activity has to be high enough to ensure that essentially all non-microbial ATP is destroyed, but if it is too high it will tend to destroy microbial ATP during step 3 above, before the ATP has the chance to generate any light. Normally the other requirements for the ATPase reagent are that it contains sufficient buffering capacity to bring the reaction mixture to a pH near the optimum of the ATPase enzyme, and that stability of the ATPase is acceptable.

### Summary of the Invention

We have discovered that the composition of the ATPase reagent in such assays can have a remarkable effect on the microbial detection properties of the overall assay. In particular, inclusion of a polyol such as sorbitol or glycerol can drastically improve the detection of certain classes of micro-organisms.

Polyols are commonly used as stabilisers for enzymes in solution. In this case, however, the action seems to be an effect on the micro-organisms in such a way that subsequent extraction of their ATP is more efficient.

According to the present invention, a method for the detection of microorganisms in a sample by bioluminescence following extraction of microbial ATP, comprises adding a polyol before or during the extraction.

This method is particularly suitable for use in the rapid detection of microbial contamination in consumer products. For example, the method can be used to test for organisms in milk and other dairy products, and provides sufficient sensitivity to allow detection of organisms that have otherwise been difficult to detect, such as *Burkholderia cepacia* and *Pseudomonas* organisms. The novel method is also useful in microbial monitoring for personal care products, non-sterile pharmaceuticals, household cleaners etc.

### Description of Preferred Embodiments

In its broadest aspect, the invention involves providing a polyol at a point in a bioluminescence assay that it can usefully enhance the sensitivity of the assay. The polyol is suitably introduced with the extractant, sequentially or simultaneously, or may be formulated as part of the extractant material. The method is thus useful in any assay conducted by bioluminescence, whether it is necessary to remove non-microbial ATP in a prior step or, as is typically the case when assaying personal care products, no such step is needed.

In an assay where it is desirable to remove non-microbial ATP, before extraction of microbial ATP, this is typically done by contacting the sample with ATPase. In this embodiment, the polyol is suitably added with the ATPase. A novel composition according to the invention comprises ATPase and a polyol. For example, an aqueous composition may comprise 0.1-5 U/ml ATPase and 5-40% polyol.

The amount of polyol that is added is typically 20% in a reagent, which is then added to an equal volume of sample. The polyol may be a hydroxylated hydrocarbon, a sugar alcohol, sugar, deoxy-sugar or amino-sugar. Representative examples are glycols such as ethylene glycol, propylene glycol, xylitol, mannitol and maltitol. Preferred polyols are sorbitol and glycerol.

### Example 1: effect of polyols on the detection of Pseudomonas aeruginosa in milk

*Pseudomonas aeruginosa* (Celsis culture collection ref. 38) was grown overnight at 30°C in UHT semi-skimmed milk, and then tested using a dairy assay protocol. The ATPase buffer was 25 mM Na-Hepes pH 7.8 containing sorbitol at 20% w/v or glycerol, ethylene glycol or propylene glycol at 20% v/v; all other reagents are available from Celsis Ltd, Cambridge, UK. The method was as follows.
1. 0.05 ml of the milk sample was transferred to a cuvette
2. 0.05 ml of the test buffer containing ATPase (LuminASE, Cat. No. 1414) was added by pipette
3. The mixture was left at room temperature for 15 minutes
4. 0.1 ml of ATP releasing agent (LuminEX, Cat. No. 92141) was added by pipette
5. The mixture was left 30 seconds for microbial ATP extraction to take place
6. 0.1 ml bioluminescence reagent (LuminATE, Cat. No. 93214 in LuminATE Buffer, Cat. No. 92158) was added automatically in the Optocomp luminometer (Celsis Ltd, Cambridge, UK)
7. After a delay of 2 seconds, the light was integrated for 4 seconds
8. The result is expressed as the mean of triplicate RLU (relative light unit) values:

| Additive (20%) | Mean RLU from P. *aeruginosa* in milk | Mean RLU relative to control |
|---|---|---|
| None | 34142 | (1.0) |
| Propylene glycol | 52512 | 1.5 |
| Ethylene glycol | 116330 | 3.4 |
| Sorbitol | 200244 | 5.9 |
| Glycerol | 359913 | 10.5 |

All of the polyols gave an increase in signal. Sorbitol and glycerol were chosen for further work.

### Example 2: effect of polyols on bioluminescence signals from ATP in milk

The results in Example 1 do not show whether the presence of polyols improves the extraction of ATP from *Pseudomonas aeruginosa,* or stimulates luciferase to give more light. In order to check this a similar experiment was performed but with no ATPase in the test buffers, and using as a sample UHT semi-skimmed milk spiked with 200 nanomolar ATP.

| Additive (20%) | Mean RLU from 200 nM ATP in milk | Mean RLU relative to control |
|---|---|---|
| None | 141787 | -1 |
| Glycerol | 135977 | 0.96 |
| Sorbitol | 147652 | 1.04 |

The presence of 20% glycerol or sorbitol has almost no effect on the bioluminescence signal from ATP.

### Example 3: detection of a panel of organisms in milk

21 different organisms from an in-house culture collection were grown overnight at 30°C in UHT semi-skimmed milk. They were then tested using a dairy assay with the ATPase dissolved in different buffers: either the normal buffer, included as part of the kit (with no polyol), or 0.2 M tris-tricine pH 7.8 + 0.005% Na azide containing 20% w/v sorbitol or 20% v/v glycerol. The cultures were (a) tested without dilution or (b) measured as actively growing organisms in milk. The latter was achieved by diluting the overnight cultures in sterile milk by a factor between 1,000 and 1,000,000 (depending on the organism) such that they were below the limit of detection, and then allowing them to grow for approx. 6 hours at 30°C before assay.

The normal dairy assay method was used, as follows.
1. 0.05 ml of the sample was transferred to a cuvette
2. Cuvettes were placed in an Advance luminometer (Celsis Ltd, Cambridge, UK)
3. 0.05 ml of the test buffer containing ATPase (LuminASE, Cat, No. 1414) was added by pipette
4. The mixture was left at room temperature for 15 minutes
5. 0.1 ml of ATP releasing agent (LuminEX, Cat. No. 92141) was injected by the luminometer
6. The mixture was left 30 seconds for microbial ATP extraction to take place
7. 0.1 ml bioluminescence reagent (LuminATE, Cat. No. 93214 in LuminATE Buffer, Cat. No. 92158) was injected by the luminometer
8. After a delay of 2 seconds, the light was integrated for 4 seconds
9. Results are expressed as the mean of triplicate RLU (relative light unit) values

The two sets of assays were run simultaneously on two luminometers in order to eliminate possible changes in the microbial populations with time. In the tables below, the reference number for each microbial species is the Celsis in-house code. The organisms are either from commercial culture collections or are isolates from contaminated products.

| Example 3(a): undiluted overnight cultures in UHT semi-skimmed milk | | | | |
|---|---|---|---|---|
| Organism | Ref | No additive | 20% glycerol | |
| | | Mean RLU | Mean RLU | RLU relative to control |
| UHT Control | - | 31 | 35 | 1.1 |
| *Bacillus cereus* | 37 | 528052 | 454082 | 0.9 |
| *Burkholderia cepacia* | 163 | 1157 | 54223 | 46.9 |
| *Candida albicans* | 10 | 8238 | 88687 | 10.8 |
| *Escherichia coli* | 45 | 438600 | 226694 | 0.5 |
| *Micrococcus luteus* | 572 | 432774 | 153789 | 0.4 |
| *Pseudomonas aeruginosa* | 38 | 1569 | 170895 | 108.9 |
| *Pseudomonas aeruginosa* | 270 | 64987 | 124610 | 1.9 |
| *Pseudomonas aeruginosa* | 316 | 44534 | 385470 | 8.7 |
| *Pseudomonas aeruginosa* | 317 | 52017 | 244622 | 4.7 |
| *Pseudomonas aeruginosa* | 318 | 37993 | 239170 | 6.3 |
| *Pseudomonas aeruginosa* | 319 | 41094 | 261965 | 6.4 |
| *Pseudomonas aeruginosa* | 341 | 26041 | 275051 | 10.6 |
| *Pseudomonas aeruginosa* | 356 | 61981 | 281733 | 4.5 |
| *Pseudomonas aeruginosa* | 423 | 144959 | 471090 | 3.2 |
| *Pseudomonas aeruginosa* | 455 | 57949 | 351372 | 6.1 |
| *Pseudomonas aeruginosa* | 539 | 16730 | 264313 | 15.8 |
| *Pseudomonas aeruginosa* | 553 | 20097 | 206392 | 10.3 |
| *Pseudomonas aeruginosa* | 554 | 35706 | 99817 | 2.8 |
| *Pseudomonas fluorescens* | 38 | 121514 | 158687 | 1.3 |
| *Pseudomonas putida* | 186 | 27315 | 193572 | 7.1 |
| *Staphylococcus aureus* | 314 | 502053 | 631309 | 1.3 |

The use of glycerol in the ATPase buffer had almost no effect on the blank signal but in most cases it caused an increase in the signal from micro-organisms. The overall result was an increase in assay sensitivity. In the panel of organisms tested, the largest improvements were shown by *Burkholderia cepacia, Candida albicans* and *Pseudomonas* species.

When actively growing cultures were assayed, a similar effect could be seen and in some cases - especially certain strains of *Pseudomonas aeruginosa -* the effect was even greater. *Burkholderia cepacia* showed a reduced effect but its detection was still improved by the use of glycerol in the ATPase buffer.

| Example 3(b): actively growing cultures in UHT semi-skimmed milk | | | | |
|---|---|---|---|---|
| Organism | Ref | No additive | 20% glycerol | |
| | | Mean RLU | Mean RLU | RLU relative to control |
| UHT Control | - | 31 | 37 | 1.2 |
| *Bacillus cereus* | 37 | 1936 | 1148 | 0.6 |
| *Burkholderia cepacia* | 163 | 454 | 925 | 2.0 |
| *Candida albicans* | 10 | 54 | 625 | 11.6 |
| *Escherichia coli* | 45 | 1096 | 1019 | 0.9 |
| *Micrococcus luteus* | 572 | 6711 | 8194 | 1.2 |
| *Pseudomonas aeruginosa* | 38 | 67 | 4855 | 72.5 |
| *Pseudomonas aeruginosa* | 270 | 273 | 4451 | 16.3 |
| *Pseudomonas aeruginosa* | 316 | 312 | 4895 | 15.7 |
| *Pseudomonas aeruginosa* | 317 | 249 | 3607 | 14.5 |
| *Pseudomonas aeruginosa* | 318 | 172 | 3953 | 23.0 |
| *Pseudomonas aeruginosa* | 319 | 140 | 4582 | 32.7 |
| *Pseudomonas aeruginosa* | 341 | 118 | 6277 | 53.2 |
| *Pseudomonas aeruginosa* | 356 | 172 | 4246 | 24.7 |
| *Pseudomonas aeruginosa* | 423 | 2018 | 6586 | 3.3 |
| *Pseudomonas aeruginosa* | 455 | 267 | 17010 | 63.7 |
| *Pseudomonas aeruginosa* | 539 | 2152 | 17082 | 7.9 |
| *Pseudomonas aeruginosa* | 553 | 189 | 4057 | 21.5 |
| *Pseudomonas aeruginosa* | 554 | 55 | 443 | 8.1 |
| *Pseudomonas fluorescens* | 38 | 303 | 2270 | 7.5 |
| *Pseudomonas putida* | 186 | 720 | 5454 | 7.6 |
| *Staphylococcus aureus* | 314 | 7322 | 14486 | 2.0 |

A parallel experiment was performed with sorbitol at 20% w/v in the ATPase buffer. This gave broadly similar results but the increases in RLU values were smaller than for glycerol.

### Example 4: detection of a panel of organisms in culture medium plus dishwashing liquid

In situations where the test samples do not contain significant amounts of non-microbial ATP, or are diluted in culture medium for an enrichment step prior to assay, it is generally not necessary to use an ATPase enzyme treatment and many commercial kits do not contain one. In example 4 we show the effect on such an assay of including, as an additional reagent, 0.2 M tris-tricine pH 7.8 + 0.005% Na azide + 20% v/v glycerol.

A sample of dishwashing liquid (DWL) was diluted 100-fold in sterile TAT broth (containing 4% Tween 20) and split into 100 ml sub-samples, which were then inoculated with low numbers of test organisms. A control sample was included which did not receive an inoculum. All samples were then incubated, shaken, for 24 hours at 30°C.

Sample were then tested with or without the use of the buffer containing 20% v/v glycerol. All other reagents are available from Celsis Ltd, Cambridge, UK. The method was as follows.
1. 0.05 ml of the sample was transferred to a cuvette
2. Cuvettes were placed in an Advance luminometer (Celsis Ltd, Cambridge, UK)
3. Where appropriate, 0.05 ml of the test buffer (containing 20% v/v glycerol) was injected by the luminometer and the mixture was left for 15 minutes
4. 0.2 ml of ATP releasing agent (LuminEX, Cat. No. 1290032) was injected by the luminometer
5. The mixture was left 10 seconds for microbial ATP extraction to take place
6. 0.1 ml bioluminescence reagent (LuminATE, Cat. No. 1290121 in LuminATE Buffer, Cat. No. 1290124) was injected by the luminometer
7. After a delay of 1 second, the light was integrated for 10 seconds
8. Results are expressed as the mean of duplicate RLU (relative light unit) values

The two sets of assays were run simultaneously on two luminometers in order to eliminate possible changes in the microbial populations with time.

| Example 4: organisms grown in TAT broth + 1% dishwashing liquid | | | | |
|---|---|---|---|---|
| Organism | Ref | No buffer | Buffer with glycerol | |
| | | Mean RLU | Mean RLU | RLU relative to control |
| None (broth only) | - | 3262 | 2148 | 0.7 |
| None (DWL in broth) | - | 2464 | 1843 | 0.7 |
| *Candida albicans* | 10 | 3930 | 93519 | 23.8 |
| *Burkholderia cepacia* | 163 | 77252 | 3679437 | 47.6 |
| *Klebsiella oxytoca* | 189 | 144826 | 5370218 | 37.1 |
| *Enterococcus faecalis* | 252 | 12931 | 201968 | 15.6 |
| *Pseudomonas aeruginosa* | 270 | 1052465 | 3837520 | 3.6 |
| *Staphylococcus aureus* | 314 | 746048 | 958809 | 1.3 |

Use of the buffer containing glycerol reduced the blank and increased the signal from micro-organisms, both of which had the effect of significantly improving assay sensitivity.

### Example 5: detection of Pseudomonas fluorescens and Burkholderia cepacia in culture medium plus different beauty, health and home samples

In order to check whether the glycerol effect would be seen in the presence of other test samples, the method of example 4 was repeated but using just 3 organisms (*Pseudomonas fluorescens* and 2 strains of *Burkholderia cepacia).* As before the organisms were grown for 24 hours in TAT broth containing a representative selection of beauty, health and home samples at a level of 1%.

| Example 5: organisms grown in TAT broth + 1% various test samples | | | | |
|---|---|---|---|---|
| Organism | Ref | No buffer | Buffer with glycerol | |
| | | Mean RLU | Mean RLU | RLU relative to control |
| No test sample (broth only) | | | | |
| None | - | 2620 | 1867 | 0.7 |
| *Pseudomonas fluorescens* | 36 | Overload | Overload | N/A |
| *Burkholderia cepacia* | 163 | 91060 | 2679131 | 29.4 |
| *Burkholderia cepacia* | 463 | 33183 | 544097 | 16.4 |

| Dishwashing liquid A | | | | |
|---|---|---|---|---|
| None | - | 2457 | 1758 | 0.7 |
| *Pseudomonas fluorescens* | 36 | 1871190 | 12794722 | 6.8 |
| *Burkholderia cepacia* | 163 | 1049092 | 3280298 | 3.1 |
| *Burkholderia cepacia* | 463 | 51735 | 1168672 | 22.6 |

| Dishwashing liquid B | | | | |
|---|---|---|---|---|
| None | - | 12205 | 8520 | 0.7 |
| *Pseudomonas fluorescens* | 36 | Overload | 10407539 | N/A |
| *Burkholderia cepacia* | 163 | 41661 | 2036908 | 48.9 |
| *Burkholderia cepacia* | 463 | 21762 | 194081 | 8.9 |

| Bodywash A | | | | |
|---|---|---|---|---|
| None | - | 5910 | 4439 | 0.8 |
| *Pseudomonas fluorescens* | 36 | Overload | Overload | N/A |
| *Burkholderia cepacia* | 163 | 45330 | 2952345 | 65.1 |
| *Burkholderia cepacia* | 463 | 371950 | 895013 | 2.4 |

| Bodywash B | | | | |
|---|---|---|---|---|
| None | - | 5613 | 4256 | 0.8 |
| *Pseudomonas fluorescens* | 36 | Overload | Overload | N/A |
| *Burkholderia cepacia* | 163 | 22591 | 824323 | 36.5 |
| *Burkholderia cepacia* | 463 | 28297 | 312657 | 11.0 |

| Toothpaste A | | | | |
|---|---|---|---|---|
| None | - | 2869 | 2204 | 0.8 |
| *Pseudomonas fluorescens* | 36 | Overload | Overload | N/A |
| *Burkholderia cepacia* | 163 | 400487 | 4599220 | 11.5 |
| *Burkholderia cepacia* | 463 | 56225 | 480552 | 8.5 |

| Toothpaste B | | | | |
|---|---|---|---|---|
| None | - | 736 | 617 | 0.8 |
| *Pseudomonas fluorescens* | 36 | 14056441 | 11526971 | 0.8 |
| *Burkholderia cepacia* | 163 | 40672 | 1272500 | 31.3 |
| *Burkholderia cepacia* | 463 | 7461 | 85278 | 11.4 |

Again, use of the buffer containing glycerol reduced the blank and in almost all cases increased the signal from micro-organisms, leading to a significant improvement in assay sensitivity.

*Pseudomonas fluorescens* grew rapidly in all samples and tended to give rise to such a high bioluminescence signal that the detection system was overloaded, in which case the RLU ratio cannot be calculated. Both strains of *Burkholderia cepacia* gave better results in the presence of the glycerol buffer.

### Example 6: effect of exposure time to glycerol buffer

This experiment was designed to test whether the 15 minutes exposure to the glycerol buffer, used in the other examples, is optimal.

2 strains of *Burkholderia cepacia* were grown overnight in tryptone soya broth and then diluted 1,000-fold in TAT broth (containing 4% Tween 20) before use. Assays, using the same reagents as Examples 4 and 5, were performed as follows.
1. 0.05 ml of the sample was transferred to a cuvette
2. 0.05 ml of the buffer with 20% v/v glycerol was added
3. After 0, 5 or 15 minutes (as appropriate) the cuvette was placed in an Optocomp luminometer
4. 0.2 ml of ATP releasing agent (LuminEX, Cat. No. 1290032) was injected by the luminometer
5. The mixture was left 10 seconds for microbial ATP extraction to take place
6. 0.1 ml bioluminescence reagent (LuminATE, Cat. No. 1290121 in LuminATE Buffer, Cat. No. 1290124) was injected by the luminometer
7. After a delay of 1 second, the light was integrated for 10 seconds
8. Results are expressed as the mean of duplicate RLU (relative light unit) values

| Organism | Ref | Time of exposure to glycerol buffer | | |
|---|---|---|---|---|
| | | 0 minutes | 5 minutes | 15 minutes |
| None (broth only) | | 2504 | 2767 | 2671 |
| *Burkholderia cepacia* | 163 | 81135 | 68711 | 66208 |
| *Burkholderia cepacia* | 463 | 51126 | 91772 | 87027 |

The results show that the exposure time of 15 minutes was not critical and an increase in assay sensitivity with the glycerol buffer occurred even when the LuminEX was added immediately after the buffer.

## Claims

1. A method for the detection of microorganisms in a sample by bioluminescence following extraction of microbial ATP, which comprises adding a polyol before or during the extraction.

2. A method according to claim 1, which additionally comprises removing non-microbial ATP, before the extraction, by contacting the sample with ATPase, wherein the polyol is added with the ATPase.

3. A method according to claim 1 or claim 2, wherein the sample is a dairy product.

4. A method according to claim 3, wherein the sample is milk.

5. A method according to any preceding claim, which additionally comprises adding a liquid nutrient medium to the sample, prior to the bioluminescence assay.

6. A method according to claim 5, wherein the sample is a household cleaner, personal care product or pharmaceutical product.

7. A method according to any preceding claim, wherein the polyol is sorbitol or glycerol.

8. A composition comprising ATPase and a polyol.
